# EUROPEAN PATENT SPECIFICATION

(11) **EP 1 294 373 B1**
(45) Date of publication and mention of the grant of the patent: **14.09.2005**
(21) Application number: 00974145.5
(22) Date of filing: 03.11.2000
(51) Int. Cl.: A61K 31/343, A61K 35/78, A61P 31/02

(54) **PHARMACEUTICAL DISINFECTANTS COMPRISING USNIC ACID AND ESSENTIAL OILS**
PHARMAZEUTISCHE DESINFEKTIONSMITTEL, DIE USNINSÄURE UND EIN ÄTHERISCHES ÖL ENTHALTEN
DESINFECTANTS PHARMACEUTIQUES CONTENANT DE L'ACIDE USNIQUE ET DES HUILES ESSENTIELLES

(30) Priority: 15.06.2000 YU 37300
(43) Date of publication of application: 26.03.2003
(73) Proprietor: AD "ZDRAVLJE" Farmaceutsko - Hemijska Industrija, Centar za Istrazivanje i Razvoj, 16000 Leskovac (YU)
(72) Inventor: STANKOVIC, Slobodan, YU-16000 Leskovac (YU); DJORDJEVIC, Ivana, YU-16000 Leskovac (YU); KOCIC, Zoran, YU-16000 Leskovac (YU)
(74) Representative: Manev, Kostadin Chanev
(86) International application number: PCT/YU2000/000022
(87) International publication number: WO 2001/095900

(56) References cited:
- EP-A- 0 256 566
- WO-A-00/03612
- DE-A- 2 354 517
- FR-A- 2 662 939
- DATABASE WPI Section Ch, Week 198942 Derwent Publications Ltd., London, GB; Class B04, AN 1989-307066 XP002164530 & RO 96 751 A (INST CERC CHIMICO-FARM), 28 April 1989 (1989-04-28)

## Description

### The field of technique

Suggested invention generally falls into the field of pharmaceutical preparations on the basis of usnic acid sodium salt and medical plants (peppermint *Mentha piperita* L.) or menthol, which are used for disinfection and for the process for their obtaining.

### Technical problem

Technical problem which is solved by this invention consists of a problem how to incorporate the active components, in this case usnic acid sodium salt (sodium salt obtained from usnic acid) and essential oils from the upper parts of medical plants (peppermint *Mentha piperita* L.) or menthol, into suitable base, which is the best way to preserve their active properties, caused by synergistic effect of above mentioned active principles and to provide simple application for disinfection of mouth and throat.

### Background of the invention

There are already known preparations for disinfection of mouth and throat, with incorporated active components containing additives (essential oils, extracts, tinctures,...), or medicinal parts of herbal drugs (peppermint)

**Peppermint** *(Mentha piperita* L.) contains up to 4% of essential oil containing minimum of 48% of menthol, menthone, menthofuran and other monoterpenes. The drug also contains up to 12% of tannins, flavonoids, triterpenes. Peppermint is a spasmolytic, carminative, diaforetic, local antiseptic, and virustatic.

Available patent and non-patent literature does not offer any similar, or any process at all, for obtaining of a pharmaceutical preparation on the basis of medical plants (sage *Salvia officinalis L*., fennel, thyme, or peppermint *Mentha piperita* L.) or menthol, and usnic acid sodium salt, which is used for disinfection of mouth and throat.

### Description of technical problem solution with examples

WO 00/03612 is related to an antimicrobial composition and its use for converting and/or stabilizing microbial perishable products as well as to microbial perishable products which contain antimicrobial compositions.

EP 0256 566 Al discloses a composition containing usnic acid or its derivatives useful for the therapeutical control of the dental caries, particularly for the preventive treatment and for the care of the cariogenic dental plaque. This compositions contain usnic acid in concentrations of between 5 mcg and 100 mg per ml or per g and include tooth pastes, mouthwashes, material for the dental medications and for the treatment of the oral cavity in several pharmaceutical and hygienic-antiseptic forms.

Usnic acid sodium salt and essential oils of medical plants (fennel *Foeniculum vulgare* L., or sage *Salvia officinalis L*., or thyme *Thymus Vulgaris L* or peppermint *Mentha piperita* L.) and menthol are strong antimicrobial and antifungal agents. The preparation obtained according to the invention comprising these active principles, showing mutual synergistic action, provides its effective use as a disinfectant for upper respiratory tract.

The essential oils built in the preparation according to the invention, show exceptionally high microbiological activity,due to the presence of certain active components.

Peppermint *Mentha piperita* L. contains: menthol, menthone, menthofuran and other monoterpenes, and their action is enhanced by presence of usnic acid sodium salt, which determines antibacterial effect of the preparation on: *Staphylococcus aureus, Echerichia coli, Salmonella sp., Bacillus subtilis, Candida*. Synergistic action of some active components of the mixture containing of usnic acid sodium salt and essential oils peppermint) or menthol - shows unexpected improvement in action on pathogenic microorganisms. Such combinations are new, and they are not comprised by the prior art, as well as the effects of the action for disinfection of the mouth and throat.

This pharmaceutical disinfective preparation on the basis of usnic acid sodium salt and medical plants (peppermint) or menthol, is used as a general disinfectant for external use and for treatment of inflammation of uper respiratory tract and oral cavity, caused by action of pathogenic microorganisms. The invention presents obtaining of the preparation in the form of: chewable tablets, tablets, gel, aerosol, as a base for deodorants, aromatic-antiseptic liquid for disinfection, in the form of wet napkin for intimate care, baby powder and other forms for mentioned purpose. The procedure for obtaining of this preparation, according to the invention, is reliable because the active components, in this case usnic acid sodium salt and essential oils preserve their natural properties, because of simplicity of the procedure for obtaining, their original antimicrobial action is neither reduced, nor lost. The advantage of the invention is in choice of active effect (usnic acid sodium salt and essential oils: peppermint) or menthol, in simplicity of the process for obtaining, as well as in simple application of the finished form of the preparation. The process used usnic acid sodium salt, obtained by a certain process from lichen *Usnea barbata L. Wigg*. This substance possesses strong antibacterial activity, particularly in oral cavity and upper respiratory tract. It is a surface antibiotic, of natural origin, which selectively inhibits carriers of an infection, not damaging healthy microflora of the mucous membrane. Peppermint essential oil contains approximately 40-80% of menthol, which is among other components of its composition one of the carriers of antimicrobial activity and aroma of the oil. Its incorporation in the preparation, according to the invention, provides a successful replacement of menthol by peppermint essential oil, not reducing the effect of the finished form of the preparation.
pharmaceutical disinfective preparation on the basis of medical plants contains usnic acid sodium salt in range 0,001 to 0,150 % by weight and peppermint Mentha piperita L. essential oil in range 0,001 to 0,400 % by weight.
pharmaceutical disinfective preparation containing usnic acid sodium salt in range 0,001 to 0.020% by weight and menthol in range 0,05 to 0,1 % by weight.

Suggested invention relates to the procedure for obtaining of pharmaceutical disinfective preparation in the form of chewable tablets, in fact, tablets for suckling, that is, solid dose forms, intended for local application of active medical substance to mucous membrane of the mouth and throat. They are mainly produced by coropressing, that is contracting, a mixture of active components and auxiliary substances.

**Chewable tablets** contain 0,001-0,020% of usnic acid sodium salt and essential oils: 0,05-0,09% of peppermint oil, or 0,03-0,08% of menthol. The procedure starts when a certain quantity of glucose is weighted into the vessel of the apparatus for preparation of a granulate. A granulating agent solution is prepared separately, by weighting a certain quantity of water into a certain vessel, heating it to 35-80□C, adding usnic acid sodium salt, certain quantity of colour and binder. Granulation of glucose is carried out with the solution prepared in this way, dispersing the solution. When inserting of the solution is over, the granulate is drying to humidity of 5-3%. Unifying of the granulate is carried out by screening through 0,5-2 mm mesh. To the cooled granulate, essential oil ( peppermint) solution is added, using sprayer for inserting. The granulate is dried to humidity which provides good tableting (max. 22%). The dried granulate is inserted into a certain vessel for homogenization, and a certain quantity of sliding agent, such as magnesium stearate, is added. The granulate prepared in such way is transferred to the tableting machine, and forming of oribletes is carried out.

The suggested invention presents the process for obtaining of pharmaceutical disinfective preparation in the form of gel, containing at least two components: the solid and the liquid ones, and also possesses the characteristics of both solids and liquids. The solid component of the gel must be colloidal and liophilic with partially solvated and usually asymmetrical particles, homogenously dispersed in a liquid medium. The liquid phase is partially bound and it fills hollows inside the spacious grid (it is immobilized).

**Gel**, containing 0,01-0,06% of usnic acid sodium salt and 0,05-0,40% of essential oils: peppermint, or menthol. The procedure consists of weighting of certain quantity of water into the vessel of the apparatus for preparation of gel, adding of usnic acid sodium salt to the water, and stirring to dissolving. Then, a certain quantity of carboxypolymethylene is added, the mixture is stirred to homogenization and left to become swollen, for 7-21 hours. To the gel base, a neutralizing agent is added slowly, with stirring, for example, sodium hydroxid solution. In a certain quantity of ethanol, dissolve the essential oil of peppermint, or menthol. This solution is gradually, with stirring added to the gel-mass to homogenization. After that, a certain quantity of colour solution is added.

The invention also presents the procedure for obtaining of a pharmaceutical disinfective preparation in the form of aerosol, which represents a suspension of liquid phase in pressure gas. As a pressure gas chlorinated or fluorized carbonhydrates (fluorotrichlormethan, difluorodichloromethan or tetrafluorodichlor). The active medical substance can have the form of solution, emulsion, powder, suspension.

**Aerosol** contains: 0,0008-0,1% of usnic acid sodium salt and 0,001-0,1% of essential oil: sage, or thyme, or fennel, or peppermint; or menthol. The procedure starts when in a certain quantity of ethanol, usnic acid sodium salt and essential oil: peppermint; or menthol. A certain quantity of water is added, as well as a certain quantity of glycerine and colour solution. To the ethanolic solution of usnic acid sodium salt and essential oil, or menthol, the aqueous solution of glycerine and colour is added. To this solution, pressure gas is added.

The invention also presents the procedure for obtaining of pharmaceutical disinfective preparation in the form of aromatic-antiseptic liquid for disinfection, which is, according to its content, alcoholic aqueous solution of active antiseptic components, sweetener, colours, and different sabilizers. It is applied as a ready liquid for application without diluting.

Aromatic-antiseptic liquid for disinfection contains: 0,01-0,15% of usnic acid sodium salt and 0,001-0,1% of essential oils: peppermint; or menthol. The process starts when in a certain quantity of water, weighed in the vessel for preparation, usnic acid sodium salt is dissolved with stirring. Into this solution, a certain quantity of glycerine and colour solution are weighed. Into weighed quantity of ethanol, the essential oil of peppermint; or menthol, is dissolved. This solution is added to the previously prepared one with stirring.

Applicability of the preparation is confirmed by determination of its microbiological activity to the following microorganisms:

The invention comprises also an application of usnic acid sodium salt in combination with the essential oil: peppermint, or menthol, according to the patent claims 1 or 2, for obtaining of pharmaceutical and cosmetic preparations for disinfection and external application.

**Table 2.**

| | |
|---|---|
| Microbiological activity of the preparation to *Bacillus subtilis* | |

| *Bacillus subtilis* | |
|---|---|
| concentration (mg/cm³) | zone diameter (mm x 10) |
| 0,0123 | 160 |
| 0,025 | 195 |
| 0,050 | 215 |
| 0,1 | 275 |
| solvent | 0 |

**Table 3.**

| | |
|---|---|
| Microbiological activity of the preparation to *Micrococcus pyogenes* | |

| *Micrococcus pyogenes* | |
|---|---|
| concentration (mg/cm³) | zone diameter (mm x 10) |
| 0,0123 | 160 |
| 0,025 | 175 |
| 0,050 | 205 |
| 0,1 | 225 |
| solvent | 0 |

**Table 4.**

| | |
|---|---|
| Microbiological activity of the preparation to *Staphylococcus aureus* | |

| *Staphylococcus aureus* | |
|---|---|
| concentration (mg/cm³) | zone diameter (mm x 10) |
| 0,0123 | 150 |
| 0,025 | 205 |
| 0,050 | 215 |
| 0,1 | 250 |
| solvent | 0 |

**Table 5.**

| | |
|---|---|
| Microbiological activity of the preparation to *Echerichia coli* | |

| *Echerichia coli* | |
|---|---|
| concentration (mg/cm³) | zone diameter (mm x 10) |
| 0,0123 | 109 |
| 0,025 | 183 |
| 0,050 | 213 |
| 0,1 | 248 |
| solvent | 0 |

**Table 6.**

| | |
|---|---|
| Microbiological activity of the preparation to *Candida albicans* | |

| *Candida albicans* | |
|---|---|
| concentration (mg/cm³) | zone diameter (mm x 10) |
| 0,0123 | 185 |
| 0,025 | 215 |
| 0,050 | 285 |
| 0,1 | 300 |
| solvent | 0 |

**Table 7.**

| | |
|---|---|
| Microbiological activity of the preparation to *Micrococcus flavus* | |

| *Micrococcus flavus* | |
|---|---|
| concentration (mg/cm³) | zone diameter (mm x 10) |
| 0,0123 | 109 |
| 0,025 | 083 |
| 0,050 | 213 |
| 0,1 | 248 |
| solvent | 0 |

The enclosed tables show that the preparation has an antibacterial effect at very low concentrations on the following microorganisms: *Bacillus subtilis, Micrococcus pyogenes, Micrococcus flavus, Echerichia coli, Staphylococcus aureus and Candida albicans*.

Following examples further illustrate, but not restrict the invention.

**Example 1.** Weigh 75,5% of glucose into the vessel for granulation. Separatelly prepare the solution of granulation agent weighing 5% of water and heat it to 60□C, adding 0,015% of usnic acid sodium salt, 0,005% of colour and 23% of binder. The solution prepared in such way is used to carry out granulation of glucose, dispersing the solution. When introducing of the solution is finished, the granulate is dried to humidity of 18%. Equalization of the granulate is carried out by screening through 2 mm mesh. To the granulate, 0,08% ethanolic solution of the peppermint essential oil is added, introducing by the sprayer. The granulate is dried to the humidity which enables good tableting (max. 22%). The dried granulate is transferred to the vessel for homogenization and 1,5% of sliding agent is added, the mixture is transferred to the tableting machine, and chewable tablets of weight up to 1200mg are formed.

**Example 2.** The procedure according to this example goes on like the procedure from example 1, the difference between these two examples is that 0,07% of the peppermint essential oil is taken. The other characteristics of the procedure are the same as those from the procedure stated in example 2.

Example 3 Weigh 96,7% of glucose into the vessel for granulation. Separatelly prepare the solution of granulation agent weighing 5% of water and heat it to 60□C, adding 0,015% of usnic acid sodium salt, 0,00587% of colour and 1,73% of binder. The solution prepared in such way is used to carry out granulation of glucose, dispersing the solution. When introducing of the solution is finished, the granulate is dried to humidity of 18%. Equalization of the granulate is carried out by screening through 2 mm mesh. The granulate is dried to the humidity which enables good tableting (max. 22%). The dried granulate is transferred to the vessel for homogenization and 1,5% of sliding agent and 0,05% of menthol is added. The prepared granulate is transferred to the tableting machine, and chewable tablets of weight up to 1200mg are formed.

### Example 4. Gel with usnic acid sodium salt and essential oils or menthol

Weigh 80,66% of water in the vessel of the apparatus for preparation, add 0,04% of usnic acid sodium salt and stir to dissolving. Subsequently add 1,0% of carboxypolymethylene, stir to homogenization (approximately 15 minutes) and leave to to become swollen, for 12 hours. To the gel base, 0,6% sodium hydroxid solution is added slowly, with stirring. Weigh 7,0% of ethanol, dissolve 0,2% solution of peppermint essential oil, or menthol. This solution is gradually, with stirring added to the gel-mass to homogenization. After that, a certain quantity of colour solution is added.

### Example 5. Aromatic-antiseptic liquid for mouth with usnic acid sodium salt and essential oils or menthol

Weigh 83,96% of water in the vessel for preparation, and 0,03% of usnic acid sodium salt is dissolved with stirring. To this solution, 5% of glycerine and 1% of colour solution are weighed. Into 10% of ethanol, 0,01% solution of the essential oil of peppermint; or menthol, is dissolved. This solution is added to the previously prepared one with stirring.

### Example 6. Aerosol for mouth with usnic acid sodium salt and essential oils or menthol

Weigh 3,5% of ethanol, and dissolve 0,0187% of usnic acid sodium salt and 0,075% solution of essential oil: peppermint; or menthol. Weigh 84,815% of water, add 7,00% of glycerine and 0,5% of colour solution. To the ethanolic solution of usnic acid sodium salt and essential oil, or menthol, the aqueous solution of glycerine and colour is added and stirred. To this solution, 50% of pressure gas, tetrafluordichlor ethan is added.

## Claims

1. A pharmaceutical disinfective preparation on the basis of medical plants, marked by the characteristic that it contains usnic acid sodium salt in range 0,001 to 0,150% by weight and peppermint *Mentha piperita* L. essential oil in range 0,001 to 0,400% by weight.

2. A pharmaceutical disinfective preparation, marked by the characteristic that it contains usnic acid sodium salt in range 0,001 to 0,020% by weight and menthol in range 0,05 to 0,1% by weight.

3. A procedure for obtaining of **chewable tablets** with 0,001-0,020% by weight of usnic acid sodium salt and 0,05-0,09% of peppermint oil or 0,03-0,08% of menthol, marked by the fact that a certain quantity of glucose is weighed, a granulation agent solution is prepared separatelly, and usnic acid sodium salt is added, the solution prepared in such manner is used to carry out granulation of glucose by dispersing the solution, the granulate is dried to humidity in range 5% to 30%, and to the cooled granulate, the essential oil ethanolic solution of: peppermint, or menthol is added, using sprayer, the granulate is dried to humidity max. 22%, to the dried granulate a certain quantity of sliding agent is added in the vessel for homogenization, and the granulate is converted to the corresponding suitable pharmaceutical form.

4. A procedure for obtaining of gels with 0.01-0.06% by weight of usnic acid sodium salt and 0.05-0.40% by weight of essential oil: peppermint, or menthol, marked by the fact that usnic acid sodium salt is dissolved in water, a certain quantity of carboxypolymethylene is added, the mixture is stirred to homogenization and left to become swollen for 7-21 hours, and then, a neutralizing agent, such as sodium hydroxid solution, is added slowly, with stirring, and subsequently, to the gel-mass the essential oil ethanolic solution of pepprmint, or menthol is added with stirring to homogenization, and finally, the colour solution is added.

5. A procedure for obtaining of aerosol with 0.0008-0.01% by weight usnic acid sodium salt and 0.001-0.1% by weight of essential oil: peppermint, or menthol, marked by the fact that in a certain quantity of ethanol usnic acid sodium salt is dissolved, as well as the essential oil of peppermint; or menthol, a certain quantity of water is added, a certain quantity of glycerine and colour solution are added as well, and to the prepared solution, pressure gas is added.

6. A procedure for obtaining of **aromatic-antiseptic liquid for disinfection** with 0,01-0,15% by weight of usnic acid sodium salt and 0,001-0,1% by weight of essential oil: peppermint, or menthol, marked by the fact that in a certain quantity of water usnic acid sodium salt is dissolved with stirring, a certain quantity of glycerine and colour solution is weighed, the essential oil ethanolic solution of: peppermint; or menthol is added with stirring to the previously prepared solution.

7. An application of usnic acid sodium salt in combination with the essential oil: peppermint, or menthol, according to the patent claims 1 or 2, for obtaining of pharmaceutical and cosmetic preparations for disinfection and external application.

## Patentansprüche

1. Pharmazeutisches desinfizierendes Präparat, **gekennzeichnet dadurch, dass** die Usninsäure des Natrium-Salzes, in Mengen von 0.001 bis 0.150 Gew.-% und die etherischen Pfefferminzeöl *(Mentha piperita L)* in Mengen, von 0.001 bis 0.400 Gew.-% enthält.

2. Pharmazeutisches desinfizierendes Präparat, **gekennzeichnet dadurch, dass** die Usninsäure des Natrium-Salzes, in Mengen von 0.001 bis 0.020 Gew.-% und eines Menthol in Mengen von 0.05% bis 0.1 Gew.% enthält.

3. Verfahren zur Herstellung von Kautabletten, von 0.001 bis 0.020 Gew.-% aus Usninsäure des Natrium-Salzes, und 0.05 - 0.09% anhand von Pfefferminzeöl, oder 0.03-0.08% anhand von Menthol der Arzneipflanzen, **gekennzeichnet dadurch, dass** eine Menge von Glucose gewogen wird, und einen granulierten Wirkstoff separat hergestellt wird und eine Usninsäure des Natriumsalzes gelöst wird, wobei die vorbereitete Lösung auf solche Weise benutzt wird, dass die Glucosegranulation durch Dispersion der Lösung ausgeführt; das Granulat wird bis zum einen Umfang von 5% bis 30% Feuchtigkeit getrocknet, und zum gekühlten Granulat, das etherische Öl, ethanolische Lösung oder Menthol bei der Anwendung von Spray zugesetzt wird; das Granulat ist bis zur max.22% Feuchtiggkeit getrocknet, während zum getrockneten Granulat eine bestimmten Mengen des gleitenden Wirkstoff den Bechälter zur Homogenisierung zugesetzt wird und das Granulat wird in eine entsprechende pharmazeutusche Form umgestellt.

4. Verfahren zur Herstellung von Gelen mit 0,01-0,06 Gew.% aus der Usninsäure vom Natrium-Salz und mit 0,05- 0,40 Gew.-% aus der etherischen Ölen oder Pfefferminze oder Menthol zugesetzt wird, **gekennzeichnet dadurch, dass** die Usninsäure vom Natrium-Salz im Wasser gelöst wird, eine gewisse Menge von Carboxypolimethylen zugesetzt wird, das Gemisch wird bis zur Homogenizirung umgerührt und daraus entsteht ein geschwollenes Gemisch, zu dem ein Neutralisierungstoff wie Natrium-Hydroxid-Lösung unter Rühren langsam zugegeben wird und bis zur Homogenisierung werden noch auch unter Rühren etherische Öle, Ethanol-Lösung von Pfefferminze oder Menthol und ausschliessend eine Farblösung zugesetzt.

5. Verfahren zur Herstellung von Spray mit 0,0008-0,01 Gew.% aus der Usninsäure von Natrim-Salz und 0,001- 0,1 Gew.-% aus den etherischen Ölen: Pfefferminze, oder Menthol, **gekennzeichnet dadurch, dass** eine gewisse Menge von Ethanol der Usninsäure des Natrium-Salzes gelöst wird, dabei etherisches Öl aus Menthol und eine gewisse Wassermenge zugesetzt werden und eine gewisse Menge vom Glyzerin und Farblösung zugesetzt werden und zu der vorbereiteten Druckgaslösung wird zugestzt.

6. Verfahren zur Herstellung vom aromatischer antiseptischer Flussigkeit zur Desinfizierung mit 0,01-0,15 Gew.-% aus der Usninsäure des Natrium-Salzes und 0,001-0,1 Gew.-% etherischen Öl, Pfefferminze, oder Menthol **gekennzeichnet dadurch dass** im gewissen Wassermenge die Usninsäure des Natrium-Salzes unter Rühren aufgelöst wird, dabei eine gewisse Glyzerinemenge und Farblösung gewogen wird, während die etherische Öl und die ethanolische Lösung der Pfefferminze, oder Menthol unter Rühren zu dem vorher vorbereiteten Lösung zugesetzt wird.

7. Awendung der Usninsäure des Natrium-Salzes in Kombination mit etherischen Ölen, Pfefferminze, oder Menthol im Einklang mit den Ansprüchen 1 oder 2 zur Herstellung von pharmazeutischen und kosmetischen Präparaten zur Desinfizierung und ausseren Anwendung.

## Revendications

1. La préparation pharmaceutique de désinfection se **caractérise par** son contenu en sel de sodium de l'acide usnic en quantité comprise entre 0,001 et 0,150 % selon le poids et en essence (huile essentielle) de la Mentha piperita L. en quantité comprise entre 0,00 et 0,400 % selon le poids.

2. La préparation pharmaceutique de désinfection se **caractérise par** son contenu en sel de sodium de l'acide usnic en quantité comprise entre 0,001 et 0,020 % selon le poids et en essence (huile essentielle) de la Mentha piperita L. et menthe en quantité comprise entre 0,05 et 0,1 % selon le poids.

3. Le procédé pour obtenir des comprimés à mâcher en sel de sodium de l'acide usnic en quantité comprise entre 0,001 et 0,020 % selon le poids et en huile de menthe en quantité comprise entre 0,05 et 0,09 % selon le poids ou bien en menthol produit à base de plantes médicinales en quantité comprise entre 0,03 et 0,08 % selon le poid, (est indique comme suit: on mesure une certaine quantité de glucose; on prepare separement une solution d'agent granulaire et on y ajoute du sel de sodium d'acide usnic. La solution ainsi obtenue est appliqué afin d'obtenir une granulation du glucose par aspersion de la solution; la substance granulaire est soumise au séchage jusqu'à l'obtention d'une humidité comprise entre 5% et 30%. A la substance granulaire refroidie on ajoute une solution d'éthanol de l'essence de la menthe ou du menthol par aspersion. La substance granulaire est ensuite soumise au séchage jusqu'à ce que la quantité de l'humidité atteint 22 % au maximum. A la substance granulaire séchée on ajoute une quantité définie de lubrifiant, le tout mis dans un récipient à homogénéisation; la substance granulaire ainsi préparé est mise dans une forme pharmaceutique appropriée.

4. Le procédé pour obtenir des gels en sel de sodium de l'acide usnic en quantité comprise entre 0,01 et 0,06 % selon le poids et en menthe ou menthol en quantité comprise entre 0,05 et 0,40 % selon le poids, se **caractérise par le fait que** le sel du sodium de l'acide usnic est dissout dans l'eau à laquelle on a ajouté une quantité déterminée de carboxypolyméthylène; le mélange se fait jusqu'à l'homogénéisation. On laise la préparation gonfler pendant 7 à 21 heures, puis, en la remuant lentement on ajoute un produit de neutralisation, tel la solution de l'hydroxyde de sodium; ensuite on ajoute, toujours en mélangeant, à cette masse en gel une solution d'éthanol d'essence de la menthe ou de menthol jusqu'à l'obtention de la homogénéisation voulue; à la fin, on ajoute une solution de substance colorante.

5. Le procédé pour obtenir des aérosols en sel de sodium de l'acide usnic en quantité comprise entre 0,0008 et 0,01 % selon le poids et en essence de la menthe ou menthol en quantité comprise entre 0,001 et 0,1 % selon le poids se **caractérise par le fait que** dans une quantité déterminée d'éthanol est dissout le sel du sodium de l'acide usnic et l'essence de la menthe ou du menthol, à quoi est ajouté une quantité convenable d'eau, de glycérine et de solution d'un colorant; à la solution ainsi préparée on ajoute de gaz sous pression.

6. Le procédé pour obtenir un liquide aromatique et antiseptique de désinfection en sel de sodium de l'acide usnic en quantité comprise entre 0,01 et 0,15 % selon le poids et en essence de la menthe ou du menthol en quantité comprise entre 0,001 et 0,1 % selon le poids, se **caractérise par le fait que** dans une quantité déterminée d'eau on dissout en mélangeant le sel du sodium de l'acide usnic, ensuite on mesure une quantité de glycérine et de solution d'un colorant; en mélangeant dans la solution préparée auparavant on ajoute une solution d'éthanol d'essence de la menthe ou de menthol.

7. Le sel de sodium de l'acide usnic s'applique en combinaison avec l'essence de la menthe ou du menthol conformémenmt aux demandes de brevets 1 ou 2, afin d'obtenir des préparations pharmaceutiques et cosmétiques pour désinfection et usage extérieur.
